# EUROPEAN PATENT SPECIFICATION

(11) **EP 2 660 332 B1**
(45) Date of publication and mention of the grant of the patent: **22.02.2017**
(21) Application number: 12166266.2
(22) Date of filing: 01.05.2012
(51) Int. Cl.: C12Q 1/68

(54) **Positive control concept**
Positives Steuerkonzept
Concept de commande positive

(43) Date of publication of application: 06.11.2013
(73) Proprietor: F. Hoffmann-La Roche AG, 4070 Basel (CH); Roche Diagnostics GmbH, 68305 Mannheim (DE)
(72) Inventor: Roehrig, Sascha Dr., 6343 Rotkreuz (CH); Riedling, Michael Dr., 6063 Dierikon (CH); Kuehler, Leif Dr., 6280 Hochdorf (CH); Namasivayam, Vijay, 6005 Luzern (CH); Simon, Christian Dr., 8919 Rottenschwil (CH); Zimmermann, Dirk, 6300 Zug (CH)
(74) Representative: Herren, Barbara

(56) References cited:
- EP-A1- 1 479 782
- WO-A1-94/06012
- WO-A1-2005/012573
- WO-A1-2007/071365
- WO-A2-02/16648
- US-A1- 2006 115 840
- DATABASE WPI Week 201053 Thomson Scientific, London, GB; AN 2010-J97837 XP002683343, & CN 101 760 515 A (PHARMIGENE INC) 30 June 2010 (2010-06-30)
- DATABASE WPI Week 201226 Thomson Scientific, London, GB; AN 2012-D36351 XP002683344, & CN 102 367 493 A (INSPECTION&QUARANTINE TECHNOLOGY CENT) 7 March 2012 (2012-03-07)
- "EGFR PCR Kit, Version 1", 1 July 2010 (2010-07-01), Qiagen, Manchester Ltd., Skelton House, Manchester, UK page 1-19,

## Description

The present invention relates to control concepts for diagnostic nucleic acid testing using amplification. In diagnostic tests for detecting or quantitating target nucleic acids by amplification, different controls are generally used. Internal controls are control nucleic acids that are added to the reaction mixtures and allow to control for the quality of every single amplification reaction and/or to quantitate the target nucleic acid in the reaction. Furthermore, negative controls, which are devoid of target nucleic acids, are run to ensure that the reagents used are not contaminated with target nucleic acids, Finally, positive controls are also used to ensure proper functioning of the system (instrument and reagents etc). Generally, for each assay, a corresponding positive control is provided. For example, for an HIV assay, a positive control comprising HIV nucleic acids is provided. For a HBV assay, a positive control comprising HBV nucleic acids is provided. For an assay for simultaneous qualitative and quantitative detection of HIV, HBV and HCV in a single vial, a positive control comprising a mixture of HIV, HBV and HCV is provided. Thus, the positive controls comprise exactly the target nucleic acids for which the assay is designed. This necessitates separate manufacturing of positive controls for each assay. Furthermore, separate control vials for each assay have to be provided to instrument systems in which the methods for detecting or quantitating target nucleic acids are performed.

WO 02/16648 discloses methods of nucleic acid amplification with an external control. The external control is amplified with specific primers and probes. Positive and negative control tests are provided. Primer and probes which are specific for the external control are also specific for the target nucleic acid to be tested. Therefore, the external control can amplified separately as a positive control.

US2006/0115840 discloses a DNA array which comprises at least one external control probe attached to at least one grid of the array. The array is hybridized with total RNA samples and added external control nucleic acids which then hybridize to their specific probes attached to the array.

WO 94/06012 discloses an analytical apparatus for carrying out an analyte assay on a strip using antibodies specific for the analyte. The apparatus has a positive control zone and a negative control zone. The positive control zone comprises a sample of the analyte of interest and a reagent which specifically reacts with the analyte of interest, i.e. a specific antibody or antibody fragment capable of binding the analyte.

WO 2005/012573 discloses a microarray comprising immobilized probes to a single target, HBV, along with a quality control probe and a negative control probe to which HBV nuleic acid comprised in a sample is hybridized.

EP 1 479 782 discloses relates to an array based hybridization assay which is useful for determining nucleic acid sequences. The assay uses immobilized positive probes along with negative probes all directed to the same target nucleic acid, which are attached to the array and are hybridized with the target nucleic acid.

The EGFR PCR Kit Handbook Version 1 by QIAGEN discloses the use of positive or negative controls for detecting different targets (polymorphisms) on one multiwell plate. The positive and negative controls are run at the same frequency.

### General Description

A method of detecting or quantitating at least two different target nucleic acids in separate vials is provided, comprising
a) amplifying said at least two target nucleic acids, wherein said first target nucleic acid is amplified in a first vial without amplifying said second target nucleic acid, and said second target nucleic acid is amplified in a second vial without amplifying said first target nucleic acid
b) In parallel with step a), amplifying a first positive control nucleic acid in a third vial, wherein said first positive control nucleic acid is a positive control for said first target nucleic acid, and amplifying a second positive control nucleic acid in a fourth vial, wherein said second positive control nucleic acid is a positive control for said second target nucleic acid; wherein said first positive control nucleic acid and said second positive control nucleic acid are provided to the third vial and the fourth vial from a positive control vial comprising a single positive control stock solution comprising a mixture of said first positive control nucleic acid and said second positive control nucleic acid before amplification,
wherein said positive control vial is provided in a rack into an automated analyzer, and wherein said negative control vial is provided in a second rack different from the rack comprising the positive control vial, wherein in said rack comprising the positive control vial, a negative control vial is absent, and wherein said negative controls are run at a higher frequency than said positive controls, wherein said method is performed in an automated analyzer.

### Brief description of Figures

Fig 1 Workflow of sample preparation
Fig. 2 Analytical system comprising vials for detection of at least two target analytes and positive control detection.
Fig. 3 Analytical system comprising vials for detection of at least three target analytes and positive control detection.
Fig. 4 Analytical system comprising vials for detection of at least two target analytes, corresponding positive controls and negative controls.
Fig. 5 shows a thermoblock with vials.

### Detailed Description

A method of detecting or quantitating at least two different target nucleic acids in separate vials is provided, comprising
a) amplifying said at least two target nucleic acids, wherein said first target nucleic acid is amplified in a first vial without amplifying said second target nucleic acid, and said second target nucleic acid is amplified in a second vial without amplifying said first target nucleic acid
b) In parallel with step a), amplifying a first positive control nucleic acid in a third vial, wherein said first positive control nucleic acid is a positive control for said first target nucleic acid, and amplifying a second positive control nucleic acid in a fourth vial, wherein said second positive control nucleic acid is a positive control for said second target nucleic acid; wherein said first positive control nucleic acid and said second positive control nucleic acid are provided to the third vial and the fourth vial from a positive control vial comprising a single positive control stock solution comprising a mixture of said first positive control nucleic acid and said second positive control nucleic acid before amplification,
wherein said positive control vial is provided in a rack into an automated analyzer, and wherein said negative control vial is provided in a second rack different from the rack comprising the positive control vial, wherein in said rack comprising the positive control vial, a negative control vial is absent, and wherein said negative controls are run at a higher frequency than said positive controls, wherein said method is performed in an automated analyzer.

The advantage of this method is that by using a single positive control stock solution comprising a mixture of at least two different target nucleic acids, only one single positive control stock solution of positive controls is required to run individual positive controls for assays that detect or quantitate different target nucleic acids. This provides improved efficiency and cost savings for manufacturing since larger batches of positive controls can be manufactured for different test kits. It also simplifies an analytical system for detecting or quantitating target nucleic acids since a single vial comprising a mixture of positive controls can be used for detecting or quantitating different target nucleic acids.

In the above method, amplifying said at least two target nucleic acids, wherein said first target nucleic acid is amplified in a first vial without amplifying said second target nucleic acid, and said second target nucleic acid is amplified in a second vial without amplifying said first target nucleic acid is achieved by adding only primers for specifically amplifying the first target nucleic acid to the first vial. Primers for amplifying the second or any further target nucleic acid are not added to the first vial and are, thus, absent from the first vial. Amplification of the second target nucleic acid is achieved by adding only primers for specifically amplifying the second target nucleic acid to the second vial. Primers for amplifying the first or any further target nucleic acid other than the second target nucleic acid are not added to the first vial and are, thus, absent from the first vial. Likewise, only primers for amplifying the positive control for the first target nucleic acid are added to the third vial, and only primers for amplifying the positive control for the second target nucleic acid are added to the fourth vial. Primers for detecting other target nucleic acids are absent from these vials. It is understood that the same principle applies for third or fourth target nucleic acids and their respective positive control reactions. Thus, if a third target nucleic acid is amplified, it is amplified in the presence of specific primers for the third target nucleic acid and in the absence of primers specific for the first or second or any other different target nucleic acid. In a specific embodiment, primers used for one specific target nucleic acid are the same as the primers used for the corresponding positive control.

The positive controls are amplified in parallel. This means that they are amplified using the same thermal profile. In one embodiment, the first, second, third and fourth vials are vessels of one multiwell plate, and the thermoblock in which the multiwell plate is placed applies the same thermal profile to all of the vessels of the multiwell plate.

The term "detecting" as used herein relates to a qualitative test aimed at assessing the presence or absence of a target nucleic acid in a sample.

The term "quantitating" as used herein relates to the determination of the amount or concentration of a target nucleic acid present in a sample.

The term "amplifying" as used herein generally refers to the production of a plurality of nucleic acid molecules from a target nucleic acid wherein primers hybridize to specific sites on the target nucleic acid molecules in order to provide an inititation site for extension by a polymerase. Amplification can be carried out by any method generally known in the art, such as but not limited to: standard PCR, long PCR, hot start PCR, qPCR, RT-PCR and Isothermal Amplification. Other amplification reactions comprise, among others, the Ligase Chain Reaction, Polymerase Ligase Chain Reaction, Gap-LCR, Repair Chain Reaction, 3SR, NASBA, Strand Displacement Amplification (SDA), Transcription Mediated Amplification (TMA), and Qβ-amplification.

The term "different target nucleic acids" as used herein relates to different targets that are assayed. A target nucleic acid is a nucleic acid that may be present in a biological sample and that is targeted by the assay. Nucleic acids comprise RNA or DNA, double stranded or single stranded nucleic acids. In one embodiment, the nucleic acids may be human or animal or viral or microbial. The term "different target nucleic acids" as used herein, thus, relates to different targets that are assayed.

If two target nucleic acids are tested in separate vials, what is meant is that one target nucleic acid is tested in one vial but not the other and vice versa for the second target nucleic acid.

The term "vial" relates to a receptacle or tube or container or vessel for holding a liquid solution. Such vials include vials in which a reaction takes place. Such vials may also relate to vials in which solutions or mixtures for use in a reaction are stored before being transferred to a different vial in which the reaction takes place. In some embodiments, more than one vial are integrally formed. In one embodiment, vials are vessels of a multiwell plate. In another embodiment, vials are individual vials which may comprise a penetrable cap and which are held in a rack.

The term "positive control nucleic acid" as used herein relates to a control nucleic acid which is a positive control for the respective target nucleic acid which, when amplified with the same primers and probes as the target nucleic acid result in a detectable signal indicating that the reaction mixture and amplification conditions permit amplification and detection of the target nucleic acid if present. As an non-limiting example, when a method is directed to quantitating HIV, the positive control nucleic acid comprises the HIV sequence and is packaged, e.g. as an armored particle. Armored particles are known in the art and mimic the envelope of HIV. Thus, the positive control nucleic acid serves as a positive control for the target nucleic acid. The positive control is generally amplified in parallel with the target nucleic acid in a separate vial.

The term "positive control vial" as used herein relates to the vial which comprises a single positive control stock solution which comprises a mixture of at least a first and a second positive control nucleic acid. The term "single positive control stock solution " means that the first and second positive control nucleic acids are present in a common positive control stock solution, not in two different individual positive control stock solutions.

The first positive control nucleic acid and said second positive control nucleic acid are, thus, provided to the third vial and the fourth vial from one single common positive control stock solution, e.g. by pipetting using a pipettor. Thus, only one positive control stock solution is required for running different individual assays for different target nucleic acids, which simplifies the system.

In one specific embodiment, the method additionally comprises amplifying in parallel with a) and b) a negative control in a fifth vial, wherein said negative control is provided from a second single negative control stock solution comprised in a negative control vial.

The term "negative control" as used herein relates to a control in which target nucleic acids are absent. The purpose of the negative control is to test if any reagents or the instrument parts of the analyzer in which the method may be performed are contaminated with target nucleic acid.

In one embodiment, the method is performed in an automated analyzer. When the method is performed in an automated analyzer, the method has the advantage that the number of positive control vials with different contents provided to the analyzer can be reduced. Thus, less vials have to be loaded, processed and identified in the analyzer.

A biological sample is provided in the method herein described by pipetting an aliquot of the biological sample into a vial. Target nucleic acids may be released from cells or viral particles by lysis. The target nucleic acid may then be enriched prior to amplification, e.g. by solid phase separation, e.g. using magnetic particles, and in the presence of a chaotropic compound. Alternatively, the target nucleic acid may be separated from other material by target capture using an oligonucleotide which specifically hybridizes to the target nucleic acid or to poly A, and which is coupled to a solid phase, e.g. a polymer material or a glass particle. Such methods are well known in the art. Following separation, the solid phase may be washed to remove inhibitors for the amplification reaction, and the bound nucleic acids may be eluted and then subjected to amplification. The positive controls and the negative controls may be exposed to the same lysis and enrichment steps as the nucleic acids to be tested.

The term "biological sample" relates to material that can be subjected to a diagnostic assay targeting nucleic acids and is usually derived from a biological source. In some embodiments, said biological sample is derived from a human and is a body liquid. In one embodiment of the invention, the biological sample is human blood, urine, sputum, sweat, swab, pipettable stool, or spinal fluid. The biological sample may also be a tissue from which target nucleic acids may be extracted.

A "target nucleic acid" is a polymeric compound of nucleotides as known to the expert skilled in the art. "Target nucleic acid" is used herein to denote a nucleic acid in a sample which should be analyzed, i.e. the presence, non-presence and/or amount thereof in a sample should be determined. The target nucleic acid may be a genomic sequence, e.g. part of a specific gene, DNA or RNA. In other embodiments, the target nucleic acid may be viral or microbial. In a specific embodiment, the target nucleic acids may be HIV, HCV and/or HBV.

The term "aliquot" as used herein relates to portions of a liquid which are employed for testing. Aliquots are typically generated by pipetting a portion of a liquid into a vial where then further treatment is conducted. When two or more aliquots of a liquid are needed it is for example possible to aspirate a volume of that liquid and to discharge portions of that volume into two or more wells. Alternatively, only the volume of a liquid intended for a single vial is dispensed at a time.

Said positive control vial is provided in a rack, and said negative control vial is provided in a second rack different from the rack comprising the positive control vial.

Vials are provided to the analyzer by loading into the analyzer. The vials are, thus, held in a rack when they are loaded into the analyzer. Loading positive control vials and negative control vials in separate racks has the advantage that negative controls can be run at a higher frequency than positive controls. This leads to higher efficiency of handling of racks holding positive or negative control vials. Thus, in said rack comprising the positive control vial, a negative control vial is absent. In one embodiment, in said rack comprising a negative control vial, a positive control vial is absent. The absence of a negative control vial in a rack holding at least one positive control vial reduces the risk of contamination of the negative control vial.

In one embodiment, said rack comprising said positive control vial comprises at least two positive control vials, wherein all of said at least two positive control vials comprise an identical mixture of positive control nucleic acids. The term "identical" relates to the positive control nucleic acids comprising the same positive control nucleic acids, although one positive control vial may have a different concentration of the positive control nucleic acids than another positive control vial in the same rack. None of the positive control vials of this embodiment comprises a positive control nucleic acid that is not comprised in the other positive control vials of the same rack. By this, the complexity of the automated system is reduced as one rack only holds positive control vials which comprise the same positive control nucleic acids. Furthermore, this also reduces the risk of cross-contamination between vials comprising different mixtures of positive control nucleic acids.

In one embodiment, at least three different target nucleic acids are detected or quantitated, additionally comprising, in step a), amplifying a third target nucleic acid in a sixth vial without amplifying said first or second target nucleic acid, and, in step b), additionally amplifying a third positive control nucleic acid, wherein said third positive control nucleic acid is a positive control for said third target nucleic acid, wherein said third positive control nucleic acid is amplified in a seventh vial, wherein said first positive control nucleic acid, said second positive control nucleic acid and said third positive control nucleic acid are provided to the third vial, the fourth vial and the seventh vial from a positive control vial comprising a single positive control stock solution comprising a mixture of said first positive control nucleic acid , said second positive control nucleic acid and said third positive control nucleic acid before amplification. By this method, one mixture of controls can, thus, be used to perform positive control reactions for three different assays detecting or quantitating three different target nucleic acids, each individually in separate vials.

On one specific embodiment, all positive control vials comprised in said first rack comprise said positive control nucleic acids at a concentration of 1x10E5 to 1x10E8 copies / ml or of 1x10E2 to 5x10E3 copies / ml. The concentrations may also be provided in other units, such as IU/ml. The factors for converting copies / ml into IU per ml depend on the specific target and may range from 1.0 to 16. Thus, the rack comprises either positive control nucleic acid at the higher or positive control nucleic acids at the lower concentration. This reduces the risk of any cross-contamination between vials comprising positive control nucleic acid at the higher concentration and vials comprising positive control nucleic acid at the lower concentration, as specified herein. Vials with only positive control nucleic acids at the higher concentration described above are used for quantitative assays. The positive control mixtures can be used despite the fact that there are more non-amplified nucleic acids in the reaction mixtures which may affect the stringency of the reactions.

In the method hereinbefore described, the tests performed may be sorted into batches of assays which match the composition of the mixture of positive control nucleic acids in the vials of one rack.

The present application also discloses a rack comprising at least one high concentration positive control vial and/or at least one low concentration positive control vial, wherein said high concentration positive control vial comprises a mixture of at least two different positive control nucleic acids at a concentration of 1x10E5 to 1x10E8 copies / ml, and said low concentration positive control vial comprises a mixture of the same two different positive control nucleic acids at a concentration of 1x10E2 to 5x10E3 copies /ml. With this rack, a mixture of high positive control nucleic acids and/or low positive control nucleic acids can be provided to an automated analyzer for performing quantitative and/or qualitative assays of at least two different parameters in different vials. In one embodiment, one rack comprises an equal number of high positive control nucleic acids and low positive control nucleic acids. The term "high positive control nucleic acids" relates to positive control nucleic acids at a higher concentration as described herein, and "low positive control nucleic acid" relates to positive control nucleic acids at a lower concentration as described herein.

In one specific embodiment, negative control vials comprising a negative control solution are absent from said rack. The absence of a negative control vial in a rack holding at least one positive control vial reduces the risk of contamination of the negative control vial.

Also disclosed is an analytical system for detecting or quantitating at least two different target nucleic acids in separate vials. The analytical system comprises vials for target nucleic acid detection, positive control and negative control detection. In one embodiment, the vials are integrally formed. A specific embodiment of vials is vessels of a multiwell plate. The method herein described can be performed on the analytical system. In one specific embodiment, the analytical system comprises a rack comprising positive control vials as described herein. In a specific embodiment, the analytical system further comprises a rack comprising negative control vials as described herein. Specific embodiments of the components of the analytical system are described herein.

In one embodiment, the rack is used in the method described herein. The rack may also be used in an automated analyzer as described herein.

The present application also discloses a use of a mixture of at least a first positive control nucleic acid and a second positive control nucleic acid in separate positive control reactions, wherein said first control nucleic acid is amplified in the absence of amplification of said second positive control nucleic acid, and said second positive control nucleic acid is amplified in the absence of amplification of said first control nucleic acid. By this, separate positive control reactions for individual targets can be based on a single positive control stock solution.

In one embodiment, the mixture is used in the method described herein. In one specific embodiment, the mixture is a high concentration mixture or a low concentration mixture. Embodiments of high and low concentration mixtures are described hereinbefore. In one embodiment, a high concentration mixture and a low concentration mixture are used in parallel. Thus, one single mixture can be used for quantitative assays and/or for qualitative assays of different target nucleic acids in parallel.

### Example

### Sample preparation

This example describes a process for isolating and simultaneously amplifying a first, a second and a third target nucleic acid in separate vials using a single positive control stock solution comprising a mixture of HIV, HBV and HCV. The same positive control stock solution was evaluated in three individual runs with the respective assay for individually quantitating HIV, HBV or HCV.

In brief, in the depicted embodiment, sample preparation and realtime PCR is carried out simultaneously under identical conditions on the positive control stock solution comprising HIV target RNA, HCV target RNA and HBV target DNA. The following positive control stock solution and negative control stock solution were analysed (HPC: High concentration positive control; LPC: Low concentration positive control):

**Table 1**

| Control | Sample name | Target name | Target concentration |
|---|---|---|---|
| Positive control stock solution | HxV HPC | HIV-1M | 5xE5 cp/ml |
| | | HBV | 1xE7 IU/ml |
| | | HCV | 5xE6 IU/ml |
| | HxV LPC | HIV-1M | 1xE3 cp/ml |
| | | HBV | 5xE2 IU/ml |
| | | HCV | 5xE2 IU/ml |
| Negative control solution | NC | N/A | N/A |

Each respective sample (200 ul) was pipetted manually into a deep well plate. To each well 50 ul of an internal quantitation standard was manually added. For the HIV and HCV assay, an RNA serving as a quantitative control was added (6xE4 armoured particles/sample). For the quantitative HBV assay, a DNA serving as a quantitative standard was added (1xE4 lambda particles/sample). The sequence of the standard nucleic acid was identical in all cases. Suitable sequences for detecting HIV, HCV and HBV as well as sequences for a standard and for detecting the standard are disclosed in EP2426222. The same standard can be used both for qualitative and for quantitative determinations.

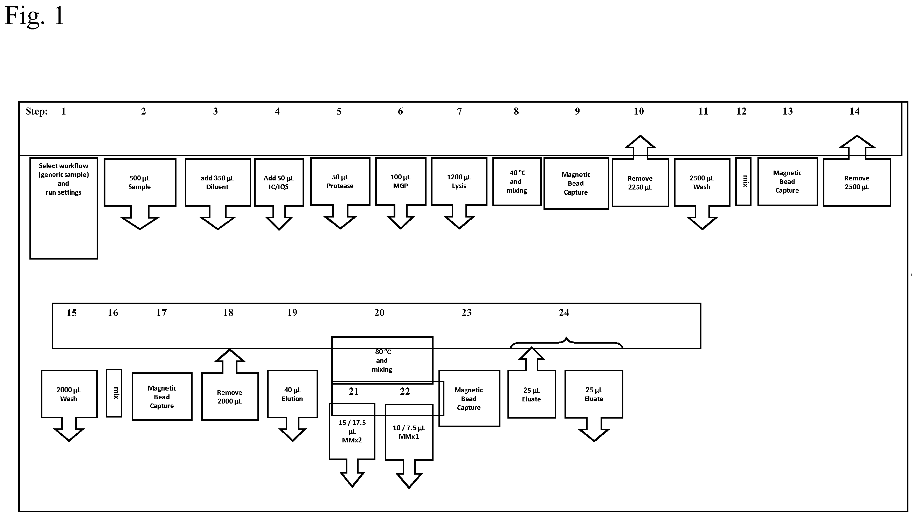

Sample preparation was performed following the workflow according to the scheme depicted in Fig. 1. In total three individual runs were performed; one run for the HIV assay, one run for the HBV assay and one run for the HCV assay. All runs were performed with the same positive control stock solution and the same negative control stock solution.

### Amplification and Detection

After the final sample preparation step of each run, the fluids containing the isolated nucleic acids were transferred to a corresponding well of a microwell plate for carrying out amplification, and the isolated nucleic acids were mixed with the respective master mixes R1 and R2 containing amplification reagents.

**Table 2 A**

| | | conc. in R1 | conc. in PCR (50uL) |
|---|---|---|---|
| R1 | MnOAc | 16.72 mM | 3.344 mM |
| | Sodium Azide | 0.09% (w/v) | 0.018 % (v/v) |
| | pH | 6.4 | |

**Table 2 B**

| HIV Assay | | conc. In R2 | conc. in PCR (50uL) |
|---|---|---|---|
| R2 | Glycerol (%, w/v) | 10% | 3% |
| | Tricine pH 8.0 | 200 mM | 60 mM |
| | DMSO (%, v/v) | 18% | 5.4% |
| | KOAc pH 7.0 | 400 mM | 120 mM |
| | Tween 20 | 0.05% | 0.015% |
| | EDTA | 146.26µM | 43.9µM |
| | Internal control forward primer | 1 µM | 0.3 µM |
| | Internal control reverse primer | 1 µM | 0.3 µM |
| | Probe internal control | 0.333 µM | 0.1 µM |
| | aptamer | 0.741 µM | 0.2222 µM |
| | ZO5D | 3000 kU/L | 0.9 U/µL (45 U/rxn) |
| | UNG | 670 kU/L | 0.2 U/µL (10 U/rxn) |
| | Sodium Azide pH 7.0 | 0.09% | 0.027% |
| | Primer 1 GAG | 1 µM | 0.3 µM |
| | Primer 2 GAG | 1 µM | 0.3 µM |
| | Probe GAG | 0.333 µM | 0.1 µM |
| | Primer2(HIV-M/-O) | 0.667 µM | 0.2 µM |
| | Primer1(HIV-M/-O) | 0.667 µM | 0.2 µM |
| | Primer2(HIV-O) | 0.167 µM | 0.05 µM |
| | Primer1(HIV-M/-O) | 0.333 µM | 0.1 µM |
| | Probe LTR | 0.333 µM | 0.1 µM |
| | dCTPs | 1333.33 µM | 400 µM |
| | dGTPs | 1333.33 µM | 400 µM |
| | dATPs | 1333.33 µM | 400 µM |
| | dUTPs | 2666.67 µM | 800 µM |
| | Final pH | 8.11 | |

**Table 2 C**

| | HCV Assay | conc. In R2 | conc. in PCR (50uL) |
|---|---|---|---|
| R2 | Glycerol (%, w/v) | 10% | 3% |
| | Tricine pH 8.0 | 200 mM | 60 mM |
| | DMSO (%, v/v) | 18% | 5.4% |
| | KOAc pH 7.0 | 400 mM | 120 mM |
| | Tween 20 | 0.05% | 0.015% |
| | EDTA | 146.26µM | 43.9µM |
| | Internal control forward primer | 1 µM | 0.3 µM |
| | Internal control reverse primer | 1 µM | 0.3 µM |
| | Probe internal control | 0.333 µM | 0.1 µM |
| | aptamer | 0.741 µM | 0.2222 µM |
| | ZO5D | 3000 kU/L | 0.9 U/µL (45 U/rxn) |
| | UNG | 670 kU/L | 0.2 U/µL (10 U/rxn) |
| | Sodium Azide pH 7.0 | 0.09% | 0.027% |
| | HCV Forward primer | 0.667 µM | 0.2 µM |
| | HCV Reverse primer | 0.333 µM | 0.1 µM |
| | HCV reverse primer | 0.667 µM | 0.2 µM |
| | HCV probe | 1 µM | 0.3 µM |
| | HCV probe | 0.333 µM | 0.1 µM |
| | dCTPs | 1333.33 µM | 400 µM |
| | dGTPs | 1333.33 µM | 400 µM |
| | dATPs | 1333.33 µM | 400 µM |
| | dUTPs | 2666.67 µM | 800 µM |
| | Final pH | 8.11 | |

**Table 2D**

| HBV Assay | | conc. In R2 | conc. in PCR (50uL) |
|---|---|---|---|
| R2 | Glycerol (%, w/v) | 10% | 3% |
| | Tricine | 200 mM | 60 mM |
| | DMSO (%, v/v) | 18% | 5.4% |
| | KOAc | 400 mM | 120 mM |
| | Tween 20 | 0.05% | 0.015% |
| | EDTA | 146.26µM | 43.9µM |
| | Internal control forward primer | 1 µM | 0.3 µM |
| | Internal control reverse primer | 1 µM | 0.3 µM |
| | Probe internal control | 0.333 µM | 0.1 µM |
| | aptamer | 0.741 µM | 0.222 µM |
| | ZO5D | 3000 kU/L | 0.9 U/µL (45 U/rxn) |
| | UNG | 670 kU/L | 0.2 U/µL (10 U/rxn) |
| | Sodium Acid | 0.09% | 0.027 |
| | HBV Forward primer | 1 µM | 0.3 µM |
| | HBV Reverse primer | 1 µM | 0.3 µM |
| | HBV Probe | 0.5 µM | 0.15 µM |
| | dCTPs | 1333.33 µM | 400 µM |
| | dGTPs | 1333.33 µM | 400 µM |
| | dATPs | 1333.33 µM | 400 µM |
| | dUTPs | 2666.67 µM | 800 µM |
| | Final pH | 8.1 | |

For amplification and detection, the microwell plate was sealed with an automated plate sealer, and the plate was transferred to an Analytical Cycler.

The following thermocycling profile was used for all assays:

**Table 2E**

| Thermo cycling profile | | | | | | |
|---|---|---|---|---|---|---|
| Program Name | Target (°C) | Acquisition Mode | Hold (hh:mm:ss) | Ramp Rate (°C/s) | Cycles | Analysis Mode |
| Pre-PCR | 50 | None | 00:02:00 | 2.2 | | |
| | 94 | None | 00:00:05 | 4.4 | | |
| | 55 | None | 00:02:00 | 2.2 | 1 | None |
| | 60 | None | 00:06:00 | 4.4 | | |
| | 65 | None | 00:04:00 | 4.4 | | |
| 1st | | | | | | |
| Measurement | 95 | None | 00:00:05 | 4.4 | 5 | Quantification |
| | 55 | Single | 00:00:30 | 2.2 | | |
| 2nd | | | | | | |
| Measurment | 91 | None | 00:00:05 | 4.4 | 45 | Quantification |
| | 58 | Single | 00:00:25 | 2.2 | | |
| Cooling | 40 | None | 00:02:00 | 2.2 | 1 | None |

The Pre-PCR program comprises initial denaturing at 94°C and incubation at 55°C, 60°C and 65°C for reverse transcription of RNA templates. Incubating at three temperatures combines the advantageous effects that at lower temperatures slightly mismatched target sequences (such as genetic variants of an organism) are also transcribed, while at higher temperatures the formation of RNA secondary structures is suppressed, thus leading to a more efficient transcription.

PCR cycling is divided into two measurements, wherein both measurements apply a one-step setup (combining annealing and extension). The first 5 cycles at 55°C allow for an increased inclusivity by pre-amplifying slightly mismatched target sequences, whereas the 45 cycles of the second measurement provide for an increased specificity by using an annealing/extension temperature of 58°C.

Using this profile on all assays mentioned above, amplification and detection was achieved for all assays and samples.

The following table 4 shows the results of HxV LPC and HxV HPC sample preparation and amplification along with the quantitation standard. It can be seen that HxV LPC and HxV HPC were successfully amplified in all cases and successfully quantitated using the quantitation standard.

**Table 4**

| | | HIV - Channel 2 | | HBV - Channel 3 | | HCV - Channel 4 | | QS - Channel 5 |
|---|---|---|---|---|---|---|---|---|
| | | Titer | CT value | Titer | CT value | Titer | CT value | CT value |
| HIV Assay | HxV LPC | 2.6E+03 cp/mL | 34.57 ± 0.34 | | | | | 36.26 ± 0.40 |
| | HxV HPC | 1.5E+06 cp/mL | 25.78 ± 0.20 | | | | | 36.29 ± 0.34 |
| | NC | N/A | N/A | | | | | 36.00 ± .046 |
| HBV Assay | HxV LPC | | | 6.5E+02 IU/mL | 30.84 ± 0.34 | | | 33.65 ± 0.16 |
| | HxV HPC | | | 1E+07 IU/mL | 16.39 ± 0.16 | | | 33.57 ± 0.14 |
| | NC | | | N/A | N/A | | | 33.49 ± 0.14 |
| HCV Assay | HxV LPC | | | | | 2.9E+02 IU/mL | 36.73 ± 0.38 | 31.09 ± 0.24 |
| | HxV HPC | | | | | 2.4E+06 IU/mL | 22.46 ± 0.20 | 30.26 ± 0.27 |
| | NC | | | | | N/A | N/A | 31.02 ± 0.37 |

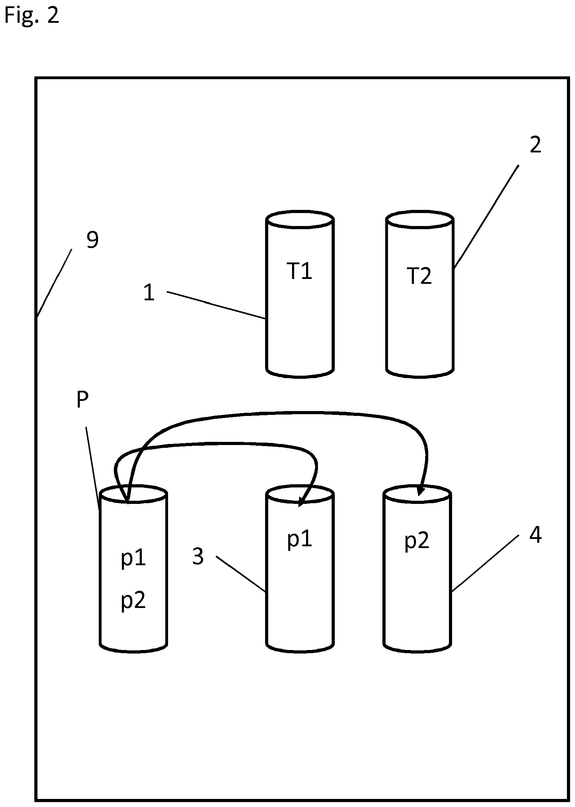

Figures 2 to 4 show exemplary embodiments of the analytical system for performing the present method. Figure 2 shows an analytical system 9. The analytical system further comprises vial 1 in which target 1 T1 is determined, vial 2 in which target 2 T2 is determined. Furthemore, it comprises vial 3 in which the positive control p1 corresponding to target 1 T1 is determined, and vial 4 in which the positive control p2 corresponding to target 2 T2 is determined. The positive controls p1 and p2 are added to the vials 3 and 4 from a positive control vial p which comprises a mixture of p1 and p2.

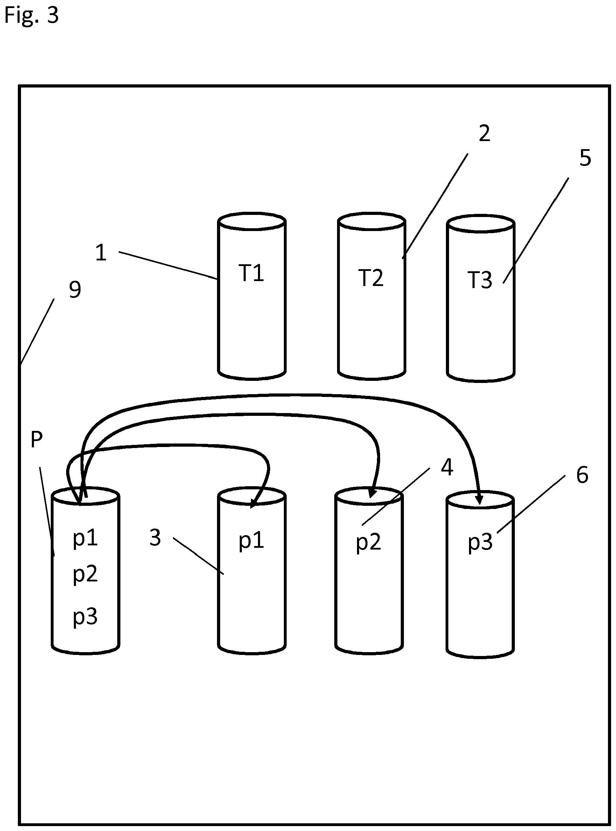

Figure 3 shows an analytical system similar to the one of Figure 2, except that three targets T1, T2 and T3 are determined, wherein the third target T3 is determined in vial 6, and three positive controls p1, p2, p3 are in the mixture of p and are distributed to vials 3, 4 and 7.

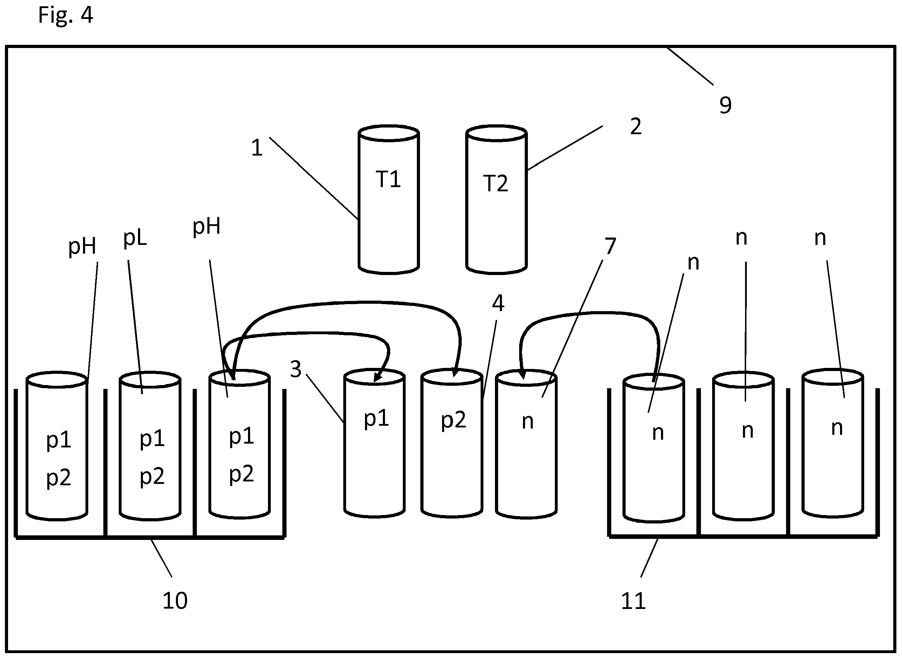

Figure 4 shows analytical system 9 in which at least two targets T1 and T2 are determined. System 9 comprises racks 10 and 11. Rack 10 comprises at least one vial pH and pL. Vial pH comprises a high concentration of positive controls, as disclosed herein, and vial pL comprises a low concentration of positive controls, as disclosed herein. Rack 11 comprises at least one vial n with a negative control. Positive controls p1 and p2 are added to vials 3 and 4 from the same positive control stock vial pH. Of course, they may also be added from the positive control stock vial PL, if a low concentration of positive controls is desired. Vials 1, 2, 3, 4 and 5 may be part of an integrally formed vessel, such as a multiwell plate, and the contents of vials 1, 2, 3, 4 and 5 are treated and reacted simultaneously and in parallel.

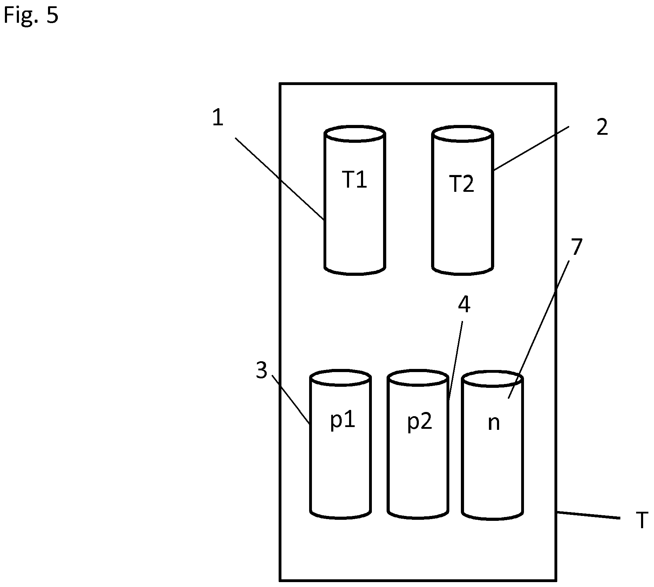

Figure 5 shows an embodiment in which vials 1, 2, 3, 4 and 7 comprising target nucleic acid T1, target nucleic acid T2, positive control p1, positive control p2 and negative control n are subjected to the same thermal profile in thermoblock T.

## Claims

1. A method of detecting or quantitating at least two different target nucleic acids in separate vials, comprising
a) amplifying said at least two target nucleic acids, wherein said first target nucleic acid is amplified in a first vial without amplifying said second target nucleic acid, and said second target nucleic acid is amplified in a second vial without amplifying said first target nucleic acid
b) in parallel with step a), amplifying a first positive control nucleic acid in a third vial, wherein said first positive control nucleic acid is a positive control for said first target nucleic acid, and amplifying a second positive control nucleic acid in a fourth vial, wherein said second positive control nucleic acid is a positive control for said second target nucleic acid; wherein said first positive control nucleic acid and said second positive control nucleic acid are provided to the third vial and the fourth vial from a positive control vial comprising a single positive control stock solution comprising a mixture of said first positive control nucleic acid and said second positive control nucleic acid before amplification,
**characterized in that**
said positive control vial is provided in a rack into an automated analyzer, and wherein said negative control vial is provided in a second rack different from the rack comprising the positive control vial, wherein in said rack comprising the positive control vial, a negative control vial is absent, and wherein said negative controls are run at a higher frequency than said positive controls, wherein said method is performed in an automated analyzer.

2. The method of claim 1, additionally comprising amplifying in parallel with a) and b) a negative control in a fifth vial, wherein said negative control is provided from a second single negative control stock solution comprised in a negative control vial.

3. The method of claim 1 or 2, wherein in said rack comprising the negative control vial, a positive control vial is absent.

4. The method of claims 1 to 3, wherein said rack comprising said positive control vial comprises at least two positive control vials, wherein all of said at least two positive control vials comprise an identical mixture of positive control nucleic acids.

5. The method of claims 1 to 4, wherein at least three different target nucleic acids are detected or quantitated, additionally comprising, in step a), amplifying a third target nucleic acid in a sixth vial without amplifying said first or second target nucleic acid, and, in step b), additionally amplifying a third positive control nucleic acid, wherein said third positive control nucleic acid is a positive control for said third target nucleic acid, wherein said third positive control nucleic acid is amplified in a seventh vial, wherein said first positive control nucleic acid, said second positive control nucleic acid and said third positive control nucleic acid are provided to the third vial, the fourth vial and the seventh vial from a positive control vial comprising a single positive control stock solution comprising a mixture of said first positive control nucleic acid , said second positive control nucleic acid and said third positive control nucleic acid before amplification.

6. The method of claims 1 to 5, wherein all positive control vials comprised in said first rack comprise said positive control nucleic acids at a concentration of 1x10E5 to 1x10E8 copies / ml or of 1x10E2 to 5x10E3 copies / ml.

## Patentansprüche

1. Verfahren zum Nachweisen oder Quantifizieren von wenigstens zwei unterschiedlichen Zielnukleinsäuren in getrennten Fläschchen, umfassend
a) Amplifizieren der wenigstens zwei Zielnukleinsäuren, wobei die erste Zielnukleinsäure in einem ersten Fläschchen ohne Amplifizieren der zweiten Zielnukleinsäure amplifiziert wird und die zweite Zielnukleinsäure in einem zweiten Fläschchen ohne Amplifizieren der ersten Zielnukleinsäure amplifiziert wird,
b) parallel zu Schritt a), Amplifizieren einer ersten Positivkontrollnukleinsäure in einem dritten Fläschchen, wobei es sich bei der ersten Positivkontrollnukleinsäure um eine Positivkontrolle für die erste Zielnukleinsäure handelt, und Amplifizieren einer zweiten Positivkontrollnukleinsäure in einem vierten Fläschchen, wobei es sich bei der zweiten Positivkontrollnukleinsäure um eine Positivkontrolle für die zweite Zielnukleinsäure handelt; wobei die erste Positiv-kontrollnukleinsäure und die zweite Positivkontrollnukleinsäure dem dritten Fläschchen und dem vierten Fläschchen vor der Amplifikation von einem Positivkontrolle-Fläschchen zugeführt werden, das eine einzelne Positivkontrolle-Stammlösung umfasst, die ein Gemisch der ersten Positivkontrollnukleinsäure und der zweiten Positivkontrollnukleinsäure umfasst,
**dadurch gekennzeichnet, dass**
das Positivkontrolle-Fläschchen in einem Gestell in einen Analyseautomaten bereitgestellt wird, und wobei das Negativkontrolle-Fläschchen in einem zweiten Gestell, das von dem das Positivkontrolle-Fläschchen umfassenden Gestell verschieden ist, bereitgestellt wird, wobei in dem das Positivkontrolle-Fläschchen umfassenden Gestell ein Negativkontrolle-Fläschchen fehlt und wobei die Negativkontrollen bei einer höheren Frequenz als die Positivkontrollen gefahren werden, wobei das Verfahren in einem Analyseautomaten erfolgt.

2. Verfahren nach Anspruch 1, zusätzlich umfassend Amplifizieren, parallel zu a) und b), einer Negativkontrolle in einem fünften Fläschchen, wobei die Negativkontrolle von einer zweiten einzelnen Negativkontrolle-Stammlösung, die in einem Negativkontrolle-Fläschchen umfasst ist, bereitgestellt wird.

3. Verfahren nach Anspruch 1 oder 2, wobei in dem das Negativkontrolle-Fläschchen umfassenden Gestell ein Positivkontrolle-Fläschchen fehlt.

4. Verfahren nach Anspruch 1 bis 3, wobei das das Positivkontrolle-Fläschchen umfassende Gestell wenigstens zwei Positivkontrolle-Fläschchen umfasst, wobei die wenigstens zwei Positivkontrolle-Fläschchen alle ein identisches Gemisch von Positivkontrollnukleinsäuren umfassen.

5. Verfahren nach Anspruch 1 bis 4, wobei wenigstens drei unterschiedliche Zielnukleinsäuren nachgewiesen oder quantifiziert werden, zusätzlich umfassend, in Schritt a), Amplifizieren einer dritten Zielnukleinsäure in einem sechsten Fläschchen ohne Amplifizieren der ersten oder zweiten Zielnukleinsäure und, in Schritt b), zusätzliches Amplifizieren einer dritten Positivkontrollnukleinsäure, wobei es sich bei der dritten Positivkontrollnukleinsäure um eine Positivkontrolle für die dritte Zielnukleinsäure handelt, wobei die dritte Positivkontrollnukleinsäure in einem siebten Fläschchen amplifiziert wird, wobei die erste Positivkontrollnukleinsäure, die zweite Positivkontrollnukleinsäure und die dritte Positivkontrollnukleinsäure dem dritten Fläschchen, dem vierten Fläschchen und dem siebten Fläschchen vor der Amplifikation von einem Positivkontrolle-Fläschchen zugeführt werden, das eine einzelne Positivkontrolle-Stammlösung umfasst, die ein Gemisch der ersten Positivkontrollnukleinsäure, der zweiten Positivkontrollnukleinsäure und der dritten Positivkontrollnukleinsäure umfasst.

6. Verfahren nach Anspruch 1 bis 5, wobei alle im ersten Gestell umfassten Positivkontrolle-Fläschchen die Positivkontrollnukleinsäuren in einer Konzentration von 1x10E5 bis 1x10E8 Kopien / ml oder von 1x10E2 bis 5x10E3 Kopien / ml umfassen.

## Revendications

1. Procédé de détection ou de quantification d'au moins deux acides nucléiques cibles différents dans des flacons séparés, comprenant
a) l'amplification desdits au moins deux acides nucléiques cibles, où ledit premier acide nucléique cible est amplifié dans un premier flacon sans amplification dudit second acide nucléique cible, et ledit second acide nucléique cible est amplifié dans un second flacon sans amplification dudit premier acide nucléique cible
b) parallèlement à l'étape a), l'amplification d'un premier acide nucléique témoin positif dans un troisième flacon, où ledit premier acide nucléique témoin positif est un témoin positif pour ledit premier acide nucléique cible, et l'amplification d'un second acide nucléique témoin positif dans un quatrième flacon, où ledit second acide nucléique témoin positif est un témoin positif pour ledit second acide nucléique cible ; où ledit premier acide nucléique témoin positif et ledit second acide nucléique témoin positif sont fournis dans le troisième flacon et le quatrième flacon à partir d'un flacon de témoins positifs contenant une unique solution mère de témoins positifs contenant un mélange dudit premier acide nucléique témoin positif et dudit second acide nucléique témoin positif avant amplification,
**caractérisé en ce que**
ledit flacon de témoins positifs est disposé dans un rack introduit dans un analyseur automatique, et ledit flacon de témoins négatifs est disposé dans un second rack différent du rack comprenant le flacon de témoins positifs, où dans ledit rack comprenant le flacon de témoins positifs, un flacon de témoins négatifs est absent, et où lesdits témoins négatifs sont analysés à une fréquence plus élevée que lesdits témoins positifs, ledit procédé étant effectué dans un analyseur automatique.

2. Procédé selon la revendication 1, comprenant en plus l'amplification, parallèlement à a) et b), d'un témoin négatif dans un cinquième flacon, où ledit témoin négatif est fourni à partir d'une unique seconde solution mère de témoins négatifs contenue dans un flacon de témoins négatifs.

3. Procédé selon la revendication 1 ou 2, où dans ledit rack comprenant le flacon de témoins négatifs, un flacon de témoins positifs est absent.

4. Procédé selon les revendications 1 à 3, où ledit rack comprenant ledit flacon de témoins positifs comprend au moins deux flacons de témoins positifs, où lesdits au moins deux flacons de témoins positifs contiennent tous un mélange identique d'acides nucléiques témoins positifs.

5. Procédé selon les revendications 1 à 4, où au moins trois acides nucléiques cibles différents sont détectés ou quantifiés, comprenant en plus, à l'étape a), l'amplification d'un troisième acide nucléique cible dans un sixième flacon sans amplification dudit premier ou second acide nucléique cible, et, à l'étape b), l'amplification supplémentaire d'un troisième acide nucléique témoin positif, où ledit troisième acide nucléique témoin positif est un témoin positif pour ledit troisième acide nucléique cible, où ledit troisième acide nucléique témoin positif est amplifié dans un septième flacon, où ledit premier acide nucléique témoin positif, ledit second acide nucléique témoin positif et ledit troisième acide nucléique témoin positif sont fournis dans le troisième flacon, le quatrième flacon et le septième flacon à partir d'un flacon de témoins positifs contenant une unique solution mère de témoins positifs contenant un mélange dudit premier acide nucléique témoin positif, dudit second acide nucléique témoin positif et dudit troisième acide nucléique témoin positif avant amplification.

6. Procédé selon les revendications 1 à 5, dans lequel tous les flacons de témoins positifs compris dans ledit premier rack contiennent lesdits acides nucléiques témoins positifs à une concentration de 1x10E5 à 1x10E8 copies/ml ou de 1x10E2 à 5x10E3 copies/ml.
